Europäisches Patentamt

European Patent Office

Office européen des brevets

(19)

(11) Publication number: **0 064 938**

**A2**

(12) # EUROPEAN PATENT APPLICATION

(21) Application number: 82730049.2

(22) Date of filing: 03.05.82

(51) Int. Cl.³: **C 12 M 1/04**

(30) Priority: 05.05.81 DE 3118285

(43) Date of publication of application:
17.11.82 Bulletin 82/46

(84) Designated Contracting States:
DE FR GB IT NL

(71) Applicant: SCHERING AKTIENGESELLSCHAFT Berlin
und Bergkamen
Müllerstrasse 170/178 Postfach 65 03 11
D-1000 Berlin 65(DE)

(72) Inventor: Müller, Rudolf, Dr.
Corneliusstrasse 17
D-1000 Berlin 46(DE)

(54) Laboratory fermentor with feed air humidification.

(57) A small fermentor system having a system for making
up evaporation losses associated therewith. The system
includes a steam feed for admixing steam with the aeration
feed to the fermentor to supersaturate the air feed with
moisture. Subsequently, the super-saturated air is passed
through means for equalizing its temperature with that of the
fermentor to condense out excess moisture. A feed conduit
then passes the moisture containing air to the fermentation
vessel. The invention also comprises a method for making
up evaporation losses.

EP 0 064 938 A2

Croydon Printing Company Ltd.

0064938

LABORATORY FERMENTOR WITH FEED
AIR HUMIDIFICATION

BACKGROUND OF THE INVENTION

This invention relates to fermentors operating under relatively high aeration rates, especially small fermentors of the type generally in use in laboratories.

In small fermentors of the type utilized in the laboratories of the chemical industry for research and development, as well as those employed at institutions of higher education, it is often necessary to maintain relatively high aeration rates during fermentation. A problem with maintaining high aeration rates is that depending on the atmospheric humidity prevailing, and on the duration of the experiment, considerable water losses from the culture broth can result. In view of the fact that losses resulting from evaporation can be determined only with great difficulty, the quantitative analytical evaluation of the fermentation processes is often beset with corresponding errors.

One proposed method of remedying this problem is by conducting the exhaust air from the fermentor through coolers, wherein the water contained in the exhaust air can be condensed, removed and recycled into the fermentor. However, even when such a procedure is employed, evaporation losses are only rarely replenished in full because these losses depend on a variety of different factors, including the ambient atmospheric humidity, the fermentor temperature, and the cooling water temperature. These variable parameters normally are not taken into account in such a system and can only be compensated for by means a complicated control system.

## OBJECTS OF THE INVENTION

It is thus an object of the invention to provide a fermentor wherein the liquid volume therein can be maintained constant independent of fermentation conditions such as time of duration of fermentation, reaction temperature, aeration rate, and relative atmospheric humidity.

It is another object of the invention to provide a fermentor capable of maintaining the liquid volume therein constant independently of fermentation conditions, and which is of simple construction.

Still another object of the invention is to provide a method of maintaining the liquid volume in a fermentor constant independent of fermentation conditions.

Upon further study of the specification and appended claims, further objects and advantages of this invention will become apparent to those skilled in the art.

## SUMMARY OF THE INVENTION

In accordance with the invention the liquid volume in the fermentor is maintained constant by means of an air supply capable of supplying feed air to a fermenting unit which is highly humidified. In one embodiment of the invention, a water circulation system is adapted for bringing the feed air temperature to the temperature prevailing in the fermentor. More specifically, a conduit is provided which brings the feed air into indirect contact with water which is circulated, at least in part in a jacket around the fermentor to equalize the temperatures of the feed air and the circulating water as discussed above.

More specifically, the air supply includes a steam feed and the admixed steam and air are fed into a temperature control vessel wherein the temperature of the feed air is equalized with that of the circulating water system. As a result, some of the moisture added by the steam is condensed but the feed air is still highly saturated with moisture so that aeration with such a feed air stream makes up for any evaporation losses in the fermentor.

The precise degree of supersaturation is not recited and of course, will vary with the steam pressure, air temperature, etc. as is well known. The steam pressure is generally 2.5 atm, and the degree of saturation is generally about 100 %.

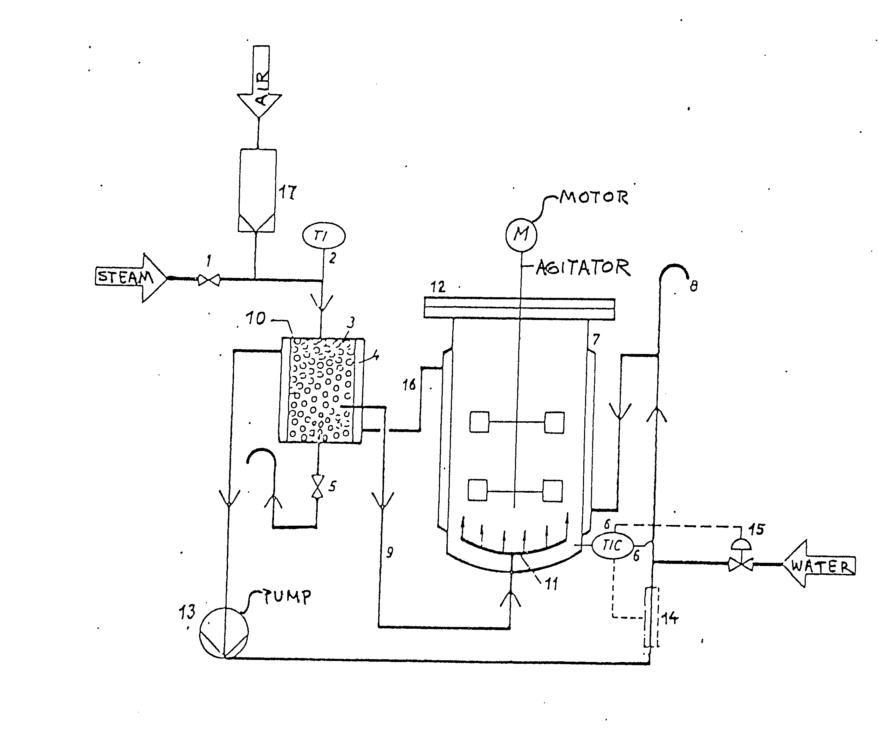

### BRIEF DESCRIPTION OF THE DRAWING

Various other objects, features and attendant advantages of the present invention will be more fully appreciated as the same becomes better understood when considered in conjunction with the accompanying drawings, in which like reference characters designate the same or similar parts throughout the several views, and wherein:

The sole figure is a schematic diagram illustrating the apparatus of the invention.

### DETAILED DISCUSSION OF THE INVENTION

In the fermentor arrangement of the invention air is fed through a filter 17. Steam is supplied through valve 1 to heat the sterilized feed air to a temperature of about 70-90°C, preferably 75 to 85 °C, which temperature is measured at thermometer TI located at a point 2 , of which the position between conjunction of steam and air stream and vessel 3 is not critical, downstream of the location wherein the sterilized air is admixed with the steam so that either more or less steam can be admixed with the air to either raise or lower the temperature. The steam supply simultaneously increases the moisture content of the sterilized feed air so that it becomes super-saturated with water, based on the fermentor temperature as will become more clearly evident from the following discussion. The steam pressure is generally **2.5 atm** which saturates the feed air **with a degree of saturation of about 100 %.** The feed air super-saturated with moisture as a result of the admixed steam is cooled in a vessel 3 to the fermentor temperature, and the excess moisture is condensed on the packing elements 10, for example glass beads or Raschig rings. The condensate from the packing 10 is then discharged through the valve 5. In operation, the valve 5 is normally adjusted so that in all cases some air is discharged with the water so that condensed water separated by this condensation is not entrained in the air feed into the fermentor 12. However, in order to ensure that there is sufficient moisture in the air fed to the fermentor 12, if no condensate is produced in element 3, the steam supply at valve 1 is increased.

In the embodiment shown, vessel 3 is located above the air outlet 11 in the fermentor 12, and air conduit 9 is arranged so that any water which may separate by condensation at a low room temperature in conduit 9 will be transferred into the fermentor 12 by means of the air stream.

The temperature in the fermentor 12 is controlled by a water circulating system which passes water through a water jacket 7 surrounding the fermentor 12. The pump 13 circulates the water, which is recovered from the jacket 7 and passed through a jacket 4 of element 3 wherein it is temperature equalized with the feed air (cf.ref. STEFANIAK et al., Indust. and Eng. Chem. $\underline{38}$ (1946)667).

Depending on the desired fermentor temperature, heating of the circulating water can be effected by means of an electrical heating unit 14, or cooling can be accomplished by merely adding cool tap water through regulating valve 15, typically a proportional control diaphragm valve. When cold water is added to the system, overflow outlet 8 serves to maintain the total volume of water circulating below a predetermined desired value. As discussed above, the water used for temperature control of the system flows through the jacket 7 of the fermentor 12 through the connecting pipe 16 into the jacket 4 of the vessel 3 and then to the pump 13. The water returning through pipe 16 is practically at the temperature of the fermentor.

With reference to the temperature control of the water in circulation, a thermostat TIC at 6 is provided and set within a predetermined range, and connected to an electric heater 14 and valve 15 for either heating the water in the system or adding more cooling water. This arrangement is **fully conventional and** will be well known to those skilled in the art.

From the foregoing description, one skilled in the art can easily ascertain the essential characteristics of this invention, and without departing from the spirit and scope thereof, can make various changes and modifications of the invention to adapt it to various usages and conditions.

WHAT IS CLAIMED IS:

1. A fermentor arrangement comprising:

a fermentation vessel having air feed means including a feed conduit associated therewith for feeding air into said vessel, said air feed means comprising a temperature control vessel having a steam feed and an air feed connected thereto for having mixed air and steam fed thereinto, said temperature control vessel also connected to said feed conduit, and having packing elements therein for condensing excess moisture from said mixed steam and air whereby moisture-containing air is fed into said fermentation vessel through said feed conduit, and a water circulating system comprising a first heat exchange jacket associated with said temperature control vessel and having connected thereto a water feed for feeding water which has been heated to the temperature of the fermentation vessel into said heat exchange jacket for substantially equalizing the temperature of the incoming air and steam passing through said packing elements with the temperature of said fermentation vessel, and pumping means connected to said heat exchange jacket for causing circulation of water in said water circulation system.

2. A fermentor arrangement according to claim 1, further comprising a separate circulation system for temperature controlling the fermentation vessel, said separate system comprising a second heat exchange jacket around the fermentation vessel for circulating water therearound, and said fermentation vessel heat exchange jacket being connected directly to said pumping means.

3. A fermentor arrangement according to claim 1, further comprising a second heat exchange jacket surrounding said fermentation vessel and connected to said first heat exchange jacket for feeding water thereinto, and said pumping means being connected to said second heat exchange jacket for pumping water thereinto.

4. A fermentor arrangement according to claim 3 further comprising a water carrying line connecting said pumping means with said second heat exchange jacket, said line having heating means and cold water supply means associated therewith by means of a temperature monitoring control system for either heating the water circulating in the circulating system or adding cold water to cool the circulating water in response to a monitoring of the temperature in said fermenting vessel.

5. A fermentor arrangement according to claim 4, further comprising discharge overflow means associated with said water circulating system for discharging excess water when cold water is added to the system.

6. A fermentor arrangement according to claim 1, further comprising discharge means connected to said temperature control vessel for discharging water condensed therein.

7.  A fermentor arrangement according to claim 1,
further comprising a control valve means for controlling
the amount of steam admixed with air entering said
temperature control vessel.

8.  A fermentor arrangement according to claim 1,
further comprising filtering means for sterilizing
incoming air prior to being admixed with steam.

9.  A fermentor arrangement according to claim 1,
further comprising temperature monitoring means for
monitoring the temperature of the incoming admixture
of steam and air.

10.  In a method of conducting a fermentation
process in a fermentation vessel wherein the contents
thereof are aerated by means of an air feed, the improvement comprising increasing the moisture content of the
air used to conduct the aeration in an amount sufficient to make up for evaporation losses occurring in
the fermentation vessel by adding steam to the feed
air to supersaturate it with moisture, and temperature
equalizing the feed air with the fermentation vessel
to condense out excess moisture not necessary to make
up evaporation losses.

0064938